(19) Europäisches Patentamt
European Patent Office
Office européen des brevets

(11) EP 0 615 447 B1

(12) **EUROPÄISCHE PATENTSCHRIFT**

(45) Veröffentlichungstag und Bekanntmachung des Hinweises auf die Patenterteilung:
**06.03.1996 Patentblatt 1996/10**

(21) Anmeldenummer: **92924552.0**

(22) Anmeldetag: **04.12.1992**

(51) Int. Cl.$^6$: **A61K 9/51**, A61K 9/10, A61K 31/19

(86) Internationale Anmeldenummer: **PCT/DE92/01015**

(87) Internationale Veröffentlichungsnummer:
**WO 93/10771 (10.06.1993 Gazette 1993/14)**

(54) **EIN FLURBIPROFEN ENTHALTENDES RETARD-ARZNEIMITTEL UND SEINE VERWENDUNG**
SLOW-RELEASE FLURBIPROFEN-CONTAINING MEDICAMENT AND ITS USE
MEDICAMENT A EFFET RETARD CONTENANT DU FLURBIPROFENE ET SON UTILISATION

(84) Benannte Vertragsstaaten:
**AT BE CH DE DK ES FR GB GR IT LI NL PT SE**

(30) Priorität: **05.12.1991 DE 4140183**

(43) Veröffentlichungstag der Anmeldung:
**21.09.1994 Patentblatt 1994/38**

(73) Patentinhaber: **ALFATEC-PHARMA GmbH**
**D-69120 Heidelberg (DE)**

(72) Erfinder:
- **WUNDERLICH, Jens-Christian**
  **D-6900 Heidelberg (DE)**
- **SCHUSTER, Otto**
  **D-6232 Bad Soden (DE)**
- **LUKAS, Helmut**
  **D-6078 Neu-Isenburg (DE)**
- **SCHICK, Ursula**
  **D-69198 Schriesheim (DE)**

(74) Vertreter: **Fürniss, Peter, Dr. et al**
**Kuhnen, Wacker & Partner**
**Alois-Steinecker-Strasse 22**
**D-85354 Freising (DE)**

(56) Entgegenhaltungen:

| | |
|---|---|
| EP-A- 0 244 118 | EP-A- 0 275 796 |
| EP-A- 0 282 020 | WO-A-91/06295 |
| FR-A- 2 608 427 | GB-A- 1 516 348 |

EP 0 615 447 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft ein Retard-Arzneimittel zur Behandlung von schmerzhaften und/oder entzündlichen, sowie fieberhaften Erkrankungen, das Flurbiprofen neben üblichen pharmazeutischen Trägern und Hilfsstoffen enthält, das dadurch gekennzeichnet ist, daß das Flurbiprofen in Form eines pharmazeutisch applizierbaren Nanosols vorliegt, das als Träger im wesentlichen Gelatine, fraktionierte Gelatine oder ein Gelatinederivat enthält.

Weiterhin betrifft die Erfindung die Verwendung eines pharmazeutisch applizierbaren Nanosols von Flurbiprofen zur Herstellung von Arzneimitteln mit analgetischer und/oder antirheumatischer Retardwirkung.

Die Erfindung betrifft schließlich ein solches Arzneimittel und seine Verwendung, bei dem das Flurbiprofen oder seine Enantiomeren teilweise als Akutform, teilweise als Retardform vorliegt.

Flurbiprofen (2-(2-Fluor-4-biphenylyl)propionsäure $C_{15}H_{13}FO_2$) der folgenden Struktur

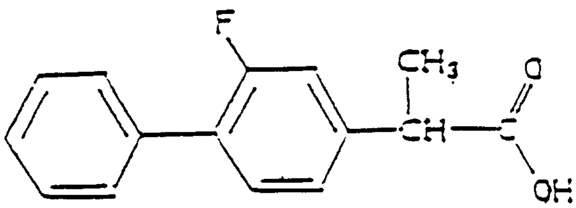

ist ein Stoff aus der Klasse der 2-Arylpropionsäuren, der sich aufgrund seines antiphlogistischen, analgetischen und antipyretischen Wirkprofils in der Therapie erfolgreich durchgesetzt hat. Handelsübliche, Flurbiprofen enthaltende Arzneimittel werden daher z.B. zur Behandlung von Schmerzen, Erkrankungen des rheumatischen Formenkreises oder Fieber eingesetzt. Tablettenzubereitungen, die 50 mg oder 100 mg Flurbiprofen enthalten, sind therapeutisch üblich, erforderliche Tagesdosen sollten, besonders bei rheumatoider Arthritis, 150-300 mg betragen.

Wie aus der chemischen Formelzeichnung und aus dem Namen ersichtlich, handelt es sich bei Flurbiprofen um ein Racemat. Dieses ist die z.Z. häufig therapeutisch angewendete Form. Wegen der Tatsache, daß jedoch unter der Therapie mit Flurbiprofen (wie auch mit anderen NSAR) unerwünschte Nebenwirkungen auftreten können, beispielsweise Irritationen des Magen-Darm-Trakts, Schleimhautperforationen, Magenulcera etc. wurde auch schon vorgeschlagen, reine Enantiomere oder Enantiomerenmischungen des Flurbiprofens einzusetzen, weil jedes Enantiomere für sich genommen ein größeres pharmakologisches Potential besitzt, als das Racemat (bei gleicher Dosierung). Dabei wurde gefunden, daß das R-Flurbiprofen, im Gegensatz zum früheren Kenntnisstand, vorwiegend für die analgetische Wirkung verantwortlich ist, während dem S-Flurbiprofen bevorzugt eine antiphlogistische Wirkung zuzuschreiben ist (DE-OS 40 28 906.0).

Es ist ebenfalls bekannt, daß die Enantiomeren des Flurbiprofens leicht durch Diastereomerentrennung mittels optisch aktiver Basen aus dem Racemat dargestellt werden können (EP 0 362 476) oder durch stereospezifische Synthese. Die enantiomerreinen Substanzen können nun beispielsweise zu einer 1:1 Mischung (Pseudoracemat) zusammengefügt werden. Solch eine Mischung des 2-Arylpropionsäurederivates Flurbiprofen, die aus je 50% des S- und des R-Enantiomeren zusammengesetzt wurde, verhält sich bezüglich ihrer Auflösungsgeschwindigkeit (Freisetzung) im wäßrigem Milieu anders als das aus je 50% S- und R-Enantiomeren bestehende, bei der Synthese anfallende racemische Flurbiprofen. Tatsächlich wurde festgestellt, daß die Auflösungsgeschwindigkeit des Pseudoracemats wesentlich höher ist, als die des Racemats.

Trotz vielfältiger galenischer Entwicklungen peroraler Retardzubereitungen ist es für Flurbiprofen bis heute noch nicht gelungen, den in-vivo Resorptionsprozeß des aus einer entsprechenden Arzneiform primär freigegebenen Wirkstoffs so optimal an die physiologischen Gegebenheiten (pH-Verhältnisse im Gastrointestinaltrakt, gastrointestinale Verweilzeiten von Formlingen, spezifische Resorptionsfenster für bestimmte Wirkstoffe) anzupassen, daß die Hauptanforderungen an eine Retardarzneiform erfüllt werden.

Retardierung eines Wirkstoffs in einer pharmazeutischen Darreichungsform ist dann erwünscht, wenn die biologische Halbwertszeit dieses Wirkstoffs kurz ist (in der Regel ca. 2 h), wobei sich Flurbiprofen mit einer Plasmahalbwertszeit von 3,9 h besonders gut eignet. Durch lang anhaltende Freigabe aus der Arzneiform im Organismus erhofft man sich verschiedene Vorteile:

1.) Verbesserte Wirkung
Möglichst genaue Einstellung von Plasmaspiegeln im therapeutischen Niveau soll einerseits Plasmaspiegelschwankungen vermindern, andererseits Nebenwirkungen (u.U. toxisch) vermeiden.

2.) Verlängerte Wirkung
damit verbunden ist analog eine entsprechende Reduktion der Einnahmehäufigkeit und dadurch eine entscheidende Erhöhung der Patienten-Compliance.

3.) Verminderung der insgesamt verabreichten Arzneistoffdosis bei Erzielung vergleichbarer Wirkung gegenüber der mehrfachen Einzelgabe.

Die galenische Konzeption von bekannten Retardarzneiformen ist i.a. so angelegt, daß die Wirkstofffreigabe im Organismus den geschwindigkeitsbestimmenden Schritt im Freigabe-Resorptions-Geschehen darstellt. Aus einem Depot soll der Wirkstoff verzögert und möglichst gleichmäßig (idealerweise konstant = Kinetik nullter Ordnung) freigegeben werden, um einen konstanten Übergang in die Biophase zu erreichen. Es ist jedoch bisher noch so, daß besagter Wirkstoff nach seiner Freisetzung weitestgehend unkontrollierbar seinem Schicksal überlassen bleibt, d.h. physiologische Einflußgrößen im Gastrointestinaltrakt bleiben unberücksichtigt.

Dies trifft besonders für die schwer wasserlösliche, schwache Wirkstoffsäure Flurbiprofen ($pK_A$ = 4,16; Schmelzpunkt des Racemats 110-111°C; Löslichkeit in Wasser 32,5 mg/l) zu, die daher zu den Wirkstoffen mit problematischer Bioverfügbarkeit gezählt werden muß. Trotz einer Vielzahl von verschiedenen galenischen Entwicklungen scheint es noch nicht gelungen zu sein, eine Formulierung für Flurbiprofen zu entwickeln, die alle Anforderungen an eine effiziente Rheuma- und/oder Schmerztherapie erfüllt.

Es ist zwar bereits vorgeschlagen worden, Flurbiprofen in vielfältig modulierbarer Art und Weise über einen bestimmten Zeitraum aus geeigneten Arzneiformen verzögert freizusetzen, es ist aber nicht anzunehmen, daß üblicherweise in-vitro gemessene Freigabe-Zeit-Profile mit der in-vivo Resorption des Flurbiprofen in Korrelation gebracht werden können. Daher gelingt es nur ungenügend, Flurbiprofen unmittelbar in resorptionsfähiger Form an den verschiedenen Resorptionsorten des Gastrointestinaltrakts zur Verfügung zu stellen. Die Folge ist nun einerseits, daß eine konstante Anflutung des Wirkstoffs in der Biophase nach Applikation einer Retardformulierung nicht sichergestellt werden kann und andererseits ein Wirkeintritt zeitlich nicht oder nur sehr schwierig vorherbestimmbar wird.

Obiges wird besonders deutlich, wenn man die gastrointestinale Passage einer konstant (nullter Ordnung) freisetzenden, Flurbiprofen enthaltenden Retardarzneiform verfolgt. Analoges gilt für die Enantiomeren S- und R-Flurbiprofen, sowie für Gemische mit unterschiedlichen Anteilen S- und R-Flurbiprofen. Nach heute allgemein akzeptierter Theorie verläuft die Wirkstoff-Resorption überwiegend nach den Gesetzen der passiven Diffusion, d.h. nur gelöste und gleichzeitig undissoziierte Wirkstoffmoleküle werden resorbiert. Die schwache, schwer wasserlösliche Wirkstoffsäure Flurbiprofen wird im Magen-Milieu (pH 1) weitestgehend undissoziiert und kristallin vorliegen. Eine nennenswerte Resorption ist daher nicht zu erwarten. Bekannte galenische Maßnahmen zur Löslichkeitserhöhung (Solubilisation, Komplexbildung) sind nicht zu empfehlen. Gleiches gilt für das Mikronisieren von Wirkstoffen, wobei eine höhere Lösungsgeschwindigkeit durch Vergrößerung der effektiven Stoffoberfläche erzwungen werden soll. Löslich gemachte Anteile können rekristallisieren, was einerseits zu Schleimhautirritationen führen kann, andererseits ist dieser Wirkstoffanteil dann endgültig für eine Magenresorption verloren.

Schwankende Magenverweilzeiten von peroralen Retardarzneiformen verhindern zusätzlich, daß eine Wirkstoffdosis einen günstigeren Resorptionsort erreicht. So ist eine Schwankungsbreite von 0,5-10 h keine Seltenheit. Nahrungsaufnahme sowie Art und Menge der Nahrung, die Größe und Dichte der Arzneiform etc. haben entscheidenden Einfluß. Die Freisetzung des Wirkstoffs läuft aber in dieser Zeit kontinuierlich weiter. Das verdeutlicht insbesondere, daß eine ausreichende Anflutung in der Biophase nicht erwartet werden kann, es resultieren subtherapeutische Plasmaspiegel. Gleichzeitig steigt das Risiko u.U. toxischer gastrointestinaler Nebenwirkungen erheblich an.

Erst nach Erreichen des oberen Dünndarms (sprunghafter pH-Anstieg) ist genügend Flurbiprofen löslich, sodaß die Resorption der undissoziierten Form in ausreichendem Maße beginnen kann. Je weiter man nun die Darmpassage verfolgt, desto höher wird der pH-Wert ansteigen (bis ca. 8). Entsprechend wird auch mehr Flurbiprofen in dissoziierter Form vorliegen und der Resorptionsprozeß mehr oder weniger stagnieren. Im Dickdarm kann darüberhinaus neben der Wirkstoffauflösung die Diffusion innerhalb des weiten Darmlumens bis zur Darmwand zu einem weiteren geschwindigkeitsbestimmenden Schritt werden. Deshalb sollte gerade in diesem Darmabschnitt die Freisetzung und Auflösung des Wirkstoffs trotz der im Vergleich zum Dünndarm geringen Flüssigkeitsströme gewährleistet sein. Dies ist bei herkömmlichen Retardformulierungen meistens nicht der Fall.

Damit wird deutlich, daß man für Flurbiprofen ein sogenanntes in-vivo Resorptionsfenster zu beachten hat. Darüberhinaus kann die Resorption auch starken interindividuellen Schwankungen unterworfen sein.

Eine diskutierte Alternative stellt das Prodrug-Konzept dar. Durch gezielte chemische Veränderung des Wirkstoffmoleküls hofft man, neue Ansätze für eine mindestens teilweise Umgehung der Resorptionsproblematik zu finden. Die tatsächliche Realisierung ist jedoch nur sehr schwierig zu bewerkstelligen. Prodrugs oder bioreversible Molekülvariationen sind neue Wirkstoffe (new-entities) deren pharmakologische und pharmakokinetische Eigenschaften im Vergleich zum ursprünglichen Wirkstoff völlig verändert sein können. Neue, aufwendige Untersuchungen hierzu werden notwendig.

Aus dem bisher Geschilderten wird klar, daß heute darüber diskutiert wird, ob der perorale Applikationsweg von Retardformen für alle Wirkstoffe überhaupt sinnvoll ist. Diese Tatsache wird besonders bei Flurbiprofen-Racemat, reinem

S- oder R-Flurbiprofen und den Mischungen aus unterschiedlichen Enantiomeranteilen deutlich. Eine Hauptvoraussetzung für die Erzielung konstanter Plasmaspiegel, nämlich die kontinuierliche Resorption von freigesetztem Wirkstoff aus der Arzneiform, ist so nicht gegeben bzw. kann mit konventioneller Galenik nicht gewährleistet werden.

J. J. Marty et al., Pharm. Acta Helv. 53, 1 (1978) S. 17-23 beschreibt die Herstellung von Gelatine-Nanopartikeln, in die auch Wirkstoffe eingeschlossen werden können. Bei der Herstellung dieser Gelatine-Nanopartikel wird zur Desolvatation und Resolvatation eine pH-Justierung vorgesehen. Eine Überführung des Arzneimittels in Nanopartikel wird nicht offenbart.

EP-A-0 282 020 beschreibt pharmazeutische Präparate in Form von Tabletten, Granulaten, Kapseln oder trockenen Sirup, bei denen der Wirkstoff in Teilchengrößen von etwa 100 µm entsprechend 100000 nm oder weniger vorliegt. Der für die Präparate verwendete Trägerstoff hat nur einen relativ geringen Anteil und stellt daher eher ein Bindemittel dar als eine echte Matrix, in die die Wirkstoffpartikel eingebettet sind.

Der vorliegenden Erfindung liegt daher die Aufgabe zugrunde, eine Arzneiform für Flurbiprofen bereitzustellen, die für retardierte Arzneistoffreigabe geeignet ist, und die die oben zum Stand der Technik genannten Nachteile weitgehend vermeidet.

Diese Aufgabe wird erfindungsgemäß durch ein Retard-Arzneimittel gemäß Patentanspruch 1 gelöst. Diese Aufgabe wird weiterhin durch die Verwendung eines pharmazeutisch applizierbaren Nanosols von Flurbiprofen gemäß den Patentansprüchen 20 und 21 gelöst.

Übliche Einzeldosierungen für das Flurbiprofen-Racemat liegen bei 200 mg bis 800 mg, im Falle des S-Flurbiprofens sind 50 mg bis 400 mg üblich. Flurbiprofen liegt im Rahmen der vorliegenden Erfindung entweder als Racemat, als racemisches Gemisch mit seinen Enantiomeren, als Pseudoracemat (Mischung von gleichen Anteilen S- und R-Flurbiprofen) oder in Mischung unterschiedlicher Anteile von S- und R-Flurbiprofen im Bereich zwischen reinem S- und reinem R-Flurbiprofen vor.

In der internationalen (PCT-) Patentanmeldung PCT/DE92/01010, auf deren Inhalt verwiesen wird, werden Nanosole und Verfahren zu ihrer Herstellung beschrieben, die es ermöglichen, kolloid-disperse Lösungen von in Wasser schwer löslichen Wirkstoffen durch Gelatine, Kollagenhydrolysate oder Gelatinederivate zu stabilisieren, wenn man den isoionischen Punkt (=Ladungsausgleich) zwischen Gelatine und den auf der Oberfläche geladenen Wirkstoffpartikeln wenigstens annähernd einstellt. Dabei bringt man das System Wirkstoffpartikel/Gelatine dadurch zum Ladungsausgleich, daß die Oberflächenladung der Partikel durch entsprechende Gegenladung der Gelatinemoleküle kompensiert wird. Erreicht wird dies durch Einstellung einer bestimmten Ladung auf den Gelatinemolekülen, die in Abhängigkeit zu ihrem isoelektrischen Punkt und dem pH-Wert der Lösung steht.

Fig. 1 zeigt eine schematische Darstellung der einstellbaren Ladungszustände von Gelatine in Abhängigkeit vom pH-Wert und IEP, wobei der IEP je nach Herstellungsart zwischen 3,5 und 9,5 liegen kann. Unterhalb von pH 3,5 sind fast alle Gelatinetypen positiv geladen. Im basischen Bereich oberhalb von pH 9,5 sind alle Gelatinetypen negativ geladen.

Fig. 2 zeigt den Mechanismus der passiven Arzneistoff-Resorption im Gastrointestinal-Trakt.

Erfindungsgemäß wird daher die Tatsache ausgenutzt, daß Gelatinen, Kollagenhydrolysate oder Gelatinederivate (nahezu unabhängig von der Viskosität) dann zu einem stabilen kolloid-dispersen Systems in Nanosolform führen, wenn der isoionische Ladungszustand zwischen Arzneistoffpartikel und Gelatine, Kollagenhydrolysat oder Gelatinederivat vorliegt.

Dagegen wurde Gelatine nach dem Stand der Technik nur zur Stabilisierung eines anorganischen, kolloid-dispersen Systems eingesetzt. So beschreibt z. B. das DAB 9 eine kolloidale Injektionslösung von radioaktivem Gold, die mit Gelatine hergestellt ist. Man stellte sich dabei lediglich vor, daß sich Makromolekül als "Kittsubstanz" zwischen den einzelnen Kolloidpartikeln befinde und so eine Teilchenaggregation verhindert werde. Dagegen war über den Stabilisierungsmechanismus, z. B. für Arzneistoffe, bisher nichts bekannt.

Die internationalen (PCT-) Patentanmeldungen vom heutigen Tage der ALFATEC-Pharma GmbH und der PAZ Arzneimittelentwicklungsgesellschaft mbH betreffen die Akutform von S- und R-Ibuprofen (PCT/DE92/01016), die Retardform von S- und R-Ibuprofen (PCT/DE92/01007), die Akutform von S- und R-Flurbiprofen (PCT/DE9201008), und die Retardform von S- und R-Flurbiprofen (PCT/DE92/01015), auf deren Inhalt verwiesen wird.

So bietet sich für die Rheumatherapie eine völlig neue Kombination aus Akut- und Retardform auf Nanosolbasis an: Akut-Flurbiprofen und Retard-Flurbiprofen.

Flurbiprofen kann erfindungsgemäß entweder als Racemat oder als racemisches Gemisch mit seinen Enantiomeren eingesetzt werden. Es eignet sich auch ein aus 50% S-Flurbiprofen und 50% R-Flurbiprofen synthetisch zusammengesetztes Pseudoracemat. Weiterhim lassen sich die reinen S- oder R-Enantiomeren einsetzen. Schließlich kann das Flurbiprofen auch als Gemisch mit unterschiedlichen Anteilen an S- und R-Enantiomeren vorliegen.

Überraschenderweise zeigt sich, daß das Vorliegen stabiler Nanopartikel im Falle des schwer wasserlöslichen Flurbiprofen-Racemats oder seiner Enantiomere völlig ausreichend ist, eine Arzneistoffresorption im gesamten Gastrointestinaltrakt (GIT) -auch im Magen - zu erreichen, die

a) unabhängig von den oben geschilderten physiologischen Bedingungen ist

b) unabhängig von den physikalisch-chemischen Eigenschaften des Flurbiprofens ist

c) nahezu vollständig ist und

d) ohne vorgelagertes Gleichgewicht der Wirkstoffauflösung erfolgt wie bei herkömmlichen Retardformen (der Wirkstoff steht in resorptionsfähiger Form unmittelbar an jedem beliebigen Resorptionsort zur Verfügung).

Offensichtlich werden Nanosole nach einem bisher nicht bekannten Mechanismus resorbiert, wie es z.B. in der Patentanmeldung "Akutform für ein Flurbiprofen enthaltendes Arzneimittel und seine Herstellung" für die überraschende Resorption im Magen in-vivo gezeigt wird.

Um die physiologischen Hintergründe der Resorption von Arzneistoffen im allgemeinen und die verbesserte Resorptionsquote der erfindungsgemäßen Nanosole ausreichend zu erläutern, ist zunächst eine Betrachtung zum Mechanismus der physiologischen Resorption von Arzneistoffen erforderlich, wie er auch in einschlägigen Publikationen dargestellt wird. Allerdings ist die vorliegende Erfindung weder an den folgenden Versuch einer wissenschaftlichen Erklärung der erfindungsgemäß auftretenden Phenomene gebunden noch kann sie hierdurch eingeschränkt werden.

Die passive Arzneistoffresorption erfolgt nach heutigem Erkenntnisstand (Theorie nach Brodie et al.), wenn folgende Bedingungen vorliegen:

a) die Gastrointestinalmembran wirkt als Lipidbarriere,

b) der Arzneistoff wird nur in gelöster und ungeladener, d.h. nichtionisierter Form aufgenommen,

c) saure Arzneistoffe werden bevorzugt im Magen, basische Arzneistoffe bevorzugt im Darm resorbiert.

Nach der peroralen Aufnahme eines Arzneistoffs in den Organismus wird seine Resorption, d.h. der Übertritt in den allgemeinen Kreislauf (Biophase) in starkem Maße durch physikalische Barrieren behindert (siehe Abb. 2), nämlich

- durch die Mucus-Schicht und eine wässerige, daran adhärierende Schicht

- die Zellmembranen der intestinalen Epithelzellen mit der daran kovalent gebundenen Glykocalix sowie

- die sogenannten "Tight Junctions", die die Epithelzellen an ihrer apikalen Seite miteinander verbinden.

Diese Barrieren bedingen, daß die Resorption von Arzneistoffen hauptsächlich abhängig von ihrem Verteilungsmechanismus und Ladungszustand- durch die Lipid-Doppelschichten erfolgt (sogenannte passive Diffusion).

Die Epithelzellen des gesamten Magen-Darm-Traktes sind mit einer Mucus-Schicht bedeckt, die aus Mucinen (Glykoproteinen), Elektrolyten, Proteinen und Nucleinsäuren besteht. Vor allem die Glykoproteine bilden mit dem Hauptanteil des Mucus, nämlich Wasser, eine viskose Gelstruktur, die in erster Linie Schutzfunktionen für die darunter liegende Epithelschicht ausübt. Die Mucusschicht ist an die apikale Oberfläche der Epithelzellen über die Glykocalix gebunden. Die Glykocalix hat ebenfalls eine Glykoproteinstruktur, die kovalent an Bausteine der Membran-Doppelschicht der Epithelzellen gebunden ist. Die verzweigten Polysaccharide der Glykocalix, die entweder direkt an amphiphile Moleküle der Doppelmembran oder an die Doppelmembran inkorporierte Proteine kovalent gebunden sind, besitzen geladene N-Acetyl-Neuraminssäure- und Sulfat-Reste und sind daher negativ geladen, was zu einer elektrostatischen Bindung oder Abstoßung von geladenen Arzneistoffmolekülen bzw. von elektrostatisch geladenen Partikeln führen kann. Die Epithelzellmembranen bestehen aus Phospholipid-Doppelschichten, in die Proteine über ihre hydrophoben Bereiche verankert sind. Die Phospholipid-Doppelschichten mit ihrem lipophilen Anteil stellen eine weitere Barriere für den Transport der zu resorbierenden Arzneistoffe dar.

Aus dieser Darstellung geht deutlich hervor, daß geladene Arzneistoffmoleküle bzw. elektrostatisch geladene Partikel daher nur eine sehr geringe Chance haben, über den peroralen Applikationsweg resorbiert zu werden.

Die erfindungsgemäßen Nanosole geben erstmalig die technische Lehre, ein System zu bilden, mit dem diese vorgenannten Resorptionshindernisse zu überwinden sind. Da die Wirkstoff-Nanopartikel durch die Gelatine erfindungsgemäß ladungsneutral stabilisiert werden, kann ihr Transport durch die negativ geladene Glykocalix ohne größere Hindernisse erfolgen, im Gegensatz zu sonstig beschriebenen Nanopartikeln des Standes der Technik, die nicht ladungsneutral stabilisiert werden bzw. stabilisiert werden können. Erfindungsgemäß kann die Einstellung des isoionischen Ladungszustandes zusätzlich noch in Abstimmung auf die physiologischen Verhältnisse erfolgen.

Da die erfindungsgemäßen Wirkstoff-Nanosole die Glykocalix ungehindert passieren können, ohne durch elektrostatische Effekte gebunden bzw. abgestoßen zu werden, erreichen sie damit auch die Oberfläche der Epithelzellen und stehen dort in hoher Konzentration zur Verfügung.

Nun können auch aktive, carriervermittelte Transportmechanismen bzw. Phagozytose einen wesentlichen Beitrag zur Resorption der Wirkstoff-Nanosole liefern.

Betrachtet man nun die gastrointestinale Passage einer geeigneten Retardformulierung, die ein Flurbiprofen-Nanosol enthält, das während dieser konstant freigegeben wird, so ergibt sich ein völlig neuartiges Bild:
Unmittelbar auf die Freigabe der Nanopartikel aus der Arzneiform im Magen erfolgt deren Resorption. Es ist dabei

gleichgültig, wie lange die Formulierung selbst im Magen verweilt. Schwankende Magenverweilzeiten von z.B. single-unit Retardarzneiformen müssen nicht mehr berücksichtigt werden, wie bei konventioneller Galenik. Damit wird ein Wirkungseintritt nach Applikation zeitlich vorhersagbar.

Eine besonders geeignete galenische Retardformulierung wird in der Patentanmeldung "Sol-gesteuerte Thermokolloidmatrix auf Gelatinebasis für perorale Retardarzneiformen" (11 AL2713) der ALFATEC-Pharma GmbH von demselben Tage vorgestellt, deren Inhalt auch zum Gegenstand der vorliegenden Erfindung gemacht wird.

Diese nahezu nullter Ordnung freisetzende Retardtablette mit einem Matrixmaterial aus Gelatine, hat sich erstaunlicherweise dadurch ausgezeichnet, daß sie die Verteilung der eingebetteten, erfindungsgemäßen Nanopartikel auf der Schleimhautoberfläche gleichmäßig gewährleistet. Somit sind die erfindungsgemäßen Nanopartikel auch wirksam vor den Einflüssen von Nahrung und Nahrungsbestandteilen geschützt.

Auch nach der Magenpassage, verbunden mit einem pH-Anstieg im oberen Dünndarm bis hin zum Dickdarm ist keine Einschränkung der Resorptionsfähigkeit der Nanopartikel in den erfindungsgemäßen Nanosolen zu befürchten. Man sollte eigentlich annehmen, daß die Stabilität der Nanosole bei pH-Werten, die oberhalb des $pK_A$-Werts von Flurbiprofen liegen, rapide absinkt bzw. die Flurbiprofen-Nanopartikel sich rasch durch Dissoziation auflösen. Dem ist jedoch nicht so.

Einerseits können die erfindungsgemäßen Nanosole durch Selektion von Gelatinesorten, die mit den Flurbiprofen-Nanopartikeln im weiter sauren Bereich, z.B. im pH-Bereich von 1,5 - 2,5 den isoionischen Punkt erreichen, hergestellt werden. Die Nanopartikel sind damit wirksam vor der Einwirkung eines schwächer sauren bis alkalischen Milieus geschützt. Die oben angesprochene, zeitliche Auflösung der Nanopartikel bei physiologischen pH-Werten, die oberhalb des $pK_A$-WertS von Flurbiprofen liegen, ist dadurch mindestens bis zur Resorption vermindert oder sogar gänzlich verhindert.

Andererseits kann durch die erfindungsgemäße Einbettung der Flurbiprofen-Nanosole in die Thermokolloidmatrix, die sich während der Gastrointestinalpassage kontrolliert auflöst, eine über mehrere Stunden nahezu konstante Resorptionsquote erzielt werden.

Denkbar als solche Matrixarzneiformen sind Matrixtabletten allein oder z.B. abgefüllt in Hartgelatinekapseln mit Akut-Flurbiprofen auf Nanosolbasis. In der Matrix selbst können auch puffernde Hilfsstoffe vorliegen, die die erfindungsgemäßen Nanopartikel bzw. das Nanosol wirksam vor physiologischen pH-Schwankungen schützen. Das die Nanopartikel bzw. Nanosol umgebende Milieu kann dadurch pH-stabil fixiert werden.

Weiterhin hat sich gezeigt, daß auch im Darm die Gelatine in den erfindungsgemäßen Nanosolen die gleichmäßige Verteilung der Nanopartikel auf der Darmschleimhaut ermöglicht. Da an der Schleimhautoberfläche, im Vergleich zum Darmlumen ein schwach saurer pH-Wert vorherrscht, sind dort die erfindungsgemäßen Nanopartikel bis zur Resorption noch effektiver vor pH-Schwankungen geschützt.

Durch alle die genannten Vorzüge gemeinsam lassen sich bei Flurbiprofen Bioverfügbarkeiten erreichen, wie sie bisher nicht bekannt sind. Damit verbunden ist ebenso eine Verkürzung der Zeit von der Applikation bis zum Erreichen der Plasmawirkstoffkonzentration im therapeutischen Nivau (steady-state), als auch eine geringe Scxhwankungsbreite des Plasmaspiegels. Außerdem wird die in der erfindungsgemäßen Arzneiform enthaltene Wirkstoffdosis vollständig ausgenutzt, sodaß damit insgesamt gesehen die analgetisch/antiphlogistische Wirkung gegenüber konventionellen Retardformen deutlich verbessert und die Verträglichkeit erhöht wird.

Erstaunlicherweise hat sich gezeigt, daß diese Nanopartikel in dem erfindungsgemäßen Nanosol an jedem gewüschten Resorptionsort ungehindert die Gastrointestinalmembran passieren können (resorbiert werden). Sie verhalten sich also, biopharmazeutisch gesehen, wie eine echte Lösung, ohne aber eine solche zu sein.

Für peroral anzuwendende Retardformen ist bisher nichts derartiges bekannt.

Da in den erfindungsgemäßen Nanosolen Teilchenwachstum durch Ostwald-Reifung wirksam vermindert wird (siehe Ausführungen der o. g. Internationalen (PCT)-Anmeldung PCT/DE92/01010 der ALFATEC-Pharma GmbH) ist keine Einschränkung der Resorptionsfähigkeit der Nanopartikel zu befürchten.

Es Hat sich weiterhin gezeigt, daß nur Nanosole, deren Größe unter 800 nm liegt, bevorzugt im Bereich von 10 nm bis 600 nm, insbesondere aber im Bereich unterhalb von 400 nm vollständig und schnell resorbiert werden können.

Efindungsgemäß können alle Gelatinessorten mit einem Maximum der Molekulargewichtsverteilung im Bereich von $10^4$ bis $10^7$ D eingesetzt werden.

Kollagenhydrolysate, fraktionierte Gelatine mit niedrigem MG, Gelatinederivate und Gelatinen mit niedrigen Bloomwerten sind dann geeignet, wenn die so hergestellten Nanosole mit geeigneten galenischen Methoden unter Zusatz von weiteren Hilfsstoffen retardiert vorliegen.

Besonders geeignet sind Gelatinesorten mit einem Peptidanteil $< 5\%$ und einem Maximum der Molekulargewichtsverteilung oberhalb von $9,5 \times 10^4$ D. Vorteilhaft können besonders hochviskose Gelatinesorten oder fraktionierte Gelatinen mit einem prozentualen Gewichtsanteil der Mikrogelfraktion ($> 10^7$ D) größer als 15 Prozent eingesetzt werden.

Solche Gelatinen besitzen in weiten pH-Bereichen eine erhöhte Pufferkapazität und fördern durch ihre hochviskose Eigenschaft die Ausbildung eines physiologischen "Nanomilieus". Sie erhöhen damit die therapeutische Wirkung und Verträglichkeit im Sinne der Erfindung.

Durch Kombination der beschriebenen Vorgehensweise lassen sich Gelatinesorten finden, die technologisch gesehen auf überraschend einfache Weise zu Retardarzneiformen mit neuen Eigenschaften führen.

Prinzipiell sind zur Herstellung der erfindungsgemäßen Nanosole die in der o. g. internationalen (PCT) Anmeldung PCT/DE92/01010 der ALFATEC-Pharma GmbH genannten Vorgehensweisen und Verfahrensvarianten geeignet, die im folgenden noch einmal angeführt werden:

Es werden mehrere Verfahren zur Herstellung der Nanosole vorgeschlagen. Dabei handelt es sich um eine beispielhafte, unvollständige Aufzählung. Der Fachmann kann aufgrund seines Fachwissens selbstständig weitere Varianten im Rahmen der vorliegenden Erfindung ausarbeiten:

**Verfahren I**

Dieses kann angewendet werden, wenn der Arzneistoff in einer Mischung aus:
einem mit Wasser mischbaren organischen Lösungsmittel und Wasser, oder
aus mehreren mit Wasser mischbaren organischen Lösungsmitteln und Wasser löslich ist:

a) eine in den Vorversuchen ausgewählte Gelatine wird mit Wasser in Solform überführt;

b) der in den Vorversuchen gefundene pH-Wert der Lösung wird eingestellt;

c) ein oder mehrere mit Wasser mischbare(s), organische(s) Lösungsmittel, vorzugsweise Ethanol, Isopropanol oder Methanol, wird/werden zu dieser Lösung gegeben;

d) der Arzneistoff wird in fester Form zu der Lösung gegeben und gelöst;

e) das/die organische(n) Lösungsmittel wird/werden entfernt, vorzugsweise durch Eindampfen in Vakuum; dabei entsteht das Nanosol;

f) die kolloid-disperse Lösung wird anschließend, vorzugsweise durch Sprüh- oder Gefriertrocknung, getrocknet.

Das organische Lösungsmittel hat die Aufgabe, den Arzneistoff zu lösen und verändert auch die Hydrathülle der Gelatinemoleküle.

**Verfahren II**

Diese Ausführungsform kann angewendet werden, wenn der Arzneistoff eine Säure oder eine Base ist, deren Salz in Wasser löslich ist:

a) eine in den Vorversuchen ausgewählte Gelatine wird mit $H_2O$ in die Solform überführt;

b) es wird ein solcher pH-Wert eingestellt, der die Salzbildung des Arzneistoffs ermöglicht;

c) der Arzneistoff wird unter Salzbildung in dem Gelatinesol gelöst;

d) durch Zugabe von Alkohol oder ähnlichen organischen Lösungsmitteln kann die Hydrathülle der Gelatinemoleküle gelockert werden;

e) durch Zugabe einer geeigneten Menge Säure oder Base wird der pH-Wert eingestellt, der zur Bildung des isoionischen Punkts (IIP) führt, dabei entsteht das Nanosol;

f) die kolloid-disperse Lösung wird wie in Verfahren I getrocknet.

Stufe d) ist fakultativ, jedoch bevorzugt.

**Verfahren III**

Diese Ausführungsform kann angewendet werden, wenn der Arzneistoff ein Neutralstoff ist:

a) es wird ein Gelatinesol hergestellt, wie unter (1) a) und b) beschrieben.

b) eine zweite Lösung aus einem mit Wasser mischbaren organischen Lösungsmittel, vorzugsweise Ethanol, Methanol, Isopropanol, Aceton und dem Arzneistoff wird hergestellt.

c) die beiden Lösungen werden vereinigt.

d) das organische Lösungsmittel wird entfernt und die kolloid-disperse Lösung wird getrocknet.

<u>Verfahren IV</u>

a) Wie unter (I) a) und b) beschrieben.

b) In einer zweiten Lösung wird ein kolloid-disperses System mit dem Arzneistoff kurzzeitig gebildet, jedoch ohne Gelatine.

c) Die unter (b) erhaltene Lösung wird kontinuierlich mit der Gelatinelösung vereinigt.

Bei Schritt (IV) c) kann die kontinuierliche Vermischung der unter (IV) a) und b) beschriebenen Lösungen zeitabhängig durch on-line Messung der Teilchengröße mit einem geeigneten Verfahren, wie z.B. durch Laser-Licht-Streuung (BI-FOQELS On-line Particle Sizer), gesteuert werden. Damit ist es möglich, eine gewünschte Partikelgröße kontinuierlich einzustellen.

Alle genannten Verfahren sind auch für Kollagenhydrolysate und Gelatinederivate geeignet und können problemlos in den technischen Maßstab übertragen werden.

Die wesentlichen Schritte können weitgehend automatisiert ablaufen, wobei auch Verfahren I bis III kontinuierlich durchführbar sind. Im Falle der Akutform für 2-Arylpropionsäurederivate seien als bevorzugt geeignete Verfahren die Varianten Nr. II und III genannt.

Für die erfindungsgemäßen Akutformen eignen sich alle Gelatinen, Gelatinederivate, Kollagenhydrolysate und fraktionierte Gelatine, sowie deren Mischungen. Gelatinesorten, die einen erfindungsgemäß beschriebenen isoelektrischen Punkt (IEP) aufweisen, der nicht handelsüblich ist, können nach den Beispielen I bis III aus o.g. deutscher Patentanmeldung hergestellt werden.

Gegenüber handelsüblichen Produkten führt die Verwendung von Gelatine, die auf spezielle Weise hergestellt wurde, zu erfindungsgemäß beschriebenen Nanosolen mit erhöhter Stabilität.

Beispiele für die Herstellung erfindungsgemäß besonders geeigneter Gelatinequalitäten werden unten gegeben.

**Beispiele für die Herstellung von erfindungsgemäß besonders geeigneten Gelatinesorten mit isoelektrischen Punkten von 3,5 bis 9,5**

Beispiel I:

**Verfahren zur Erzielung eines IEP's von 7,5 bis 9,5**

Kollagenhaltiges Ausgangsmaterial wie z.B. Schweineschwarten werden mit einer wäßrigen Lösung einer 0,45 N Mineralsäure, vorzugsweise Schwefelsäure, im Flottenverhältnis 1:1 12 bis 20 Stunden behandelt. Anschließend wird der Säureüberschuß durch mehrmaliges Waschen entfernt, wobei zur Abkürzung des Verfahrens Natriumhydrogencarbonat verwendet werden kann. Die Extraktion des sudreifen Materials erfolgt mit heißem Wasser bei 55 - 80° C bei einem pH von 2,5 bis 4,5. Bei pH-Werten unterhalb von 3,5 kann ein IEP von 8,5 bis 9,5 erreicht werden, bei pH-Werten oberhalb 3,5 liegt der IEP bei 7 bis 8,5. Auf diese Weise lassen sich verschiedene IEP's von 7 bis 9,5 in direkter Abhängigkeit vom pH-Wert während der Extraktion erzielen.

Nach der Verfahrensstufe der Extraktion wird die wäßrige Lösung neutralisiert und wie üblich aufgearbeitet.

Durch dieses Verfahren kann man weiterhin in Abhängigkeit von der gewählten Temperatur während der Extraktion Gelatinesorten mit hohen bis mittleren Molekulargewichtsverteilungen erhalten.

Bei Temperaturen von 50-55° C erhält man besonders hochviskose und hochbloomige Qualitäten. Gelatinesorten mit niedrigem Molekulargewicht bzw. kaltwasserlösliche Gelatinen können durch gezielten Abbau mit Kollagenasen erhalten werden.

Beispiel II:

**Verfahren zur Erzielung eines IEP's von 4 bis 7,5**

Das kollagenhaltige Ausgangsmaterial wird zur Entfernung von Fremdstoffen zunächst gewaschen, zerkleinert und anschließend durch Zusatz von Magnesit, Natronlauge oder Calciumhydroxid durch gründliches Vermischen im Flottenverhältnis 1:1,2 homogen alkalisch gemacht. Das so vorbehandelte Material wird kurzzeitig druckhydrolytisch bei 1,01 x $10^5$ bis 2,02 x $10^5$ Pa und einem pH-Wert der wäßrigen Lösung von 8-14 aufgeschlossen. Nach dem Aufschluß wird sofort neutralisiert und die noch heiße wäßrige Gelatinelösung wie üblich filtriert, entsalzt, aufkonzentriert und getrocknet.

Nimmt man ein schwach basisches Aufschlußmittel wie Magnesit, erhält man einen IEP von 6 bis 7,5, sofern man bei 1,01 x $10^5$ Pa arbeitet. IEP's von 5 bis 6 erhält man bei Einsatz einer verdünnten Kalkmilchsuspension und bei Verwendung von 0,005 bis 0,1 N Natronlauge können IEP's von 4 bis 5 erzielt werden.

Gelatinesorten mit geringem Racemisierungsgrad und niedrigem Peptidanteil lassen sich bei Druckverhältnissen von 1,01 x $10^5$ Pa und Verweilzeiten von maximal 10 Min. erreichen.

Mittel- bis niedrigmolekulare bis hin zu kaltwasserlöslichen Sorten ergeben sich durch entsprechend längere Verweilzeiten.

Beispiel III:

**Verfahren zur Erzielung eines IEP's von 3,5 bis 6**

Kollagenhaltiges Ausgangsmaterial, vorzugsweise Spalt bzw. Ossein, wird nach der Eingangswäsche einem Kurzzeitäscher unterworfen. Hierbei bieten sich zwei Verfahrensvarianten im Flottenverhältnis 1:1,3 an, die entweder eine gesättigte Kalkmilchsuspension oder eine 0,1 bis 1 N Natronlauge zum Einsatz bringen.

Bei Verwendung einer Kalkmilchsuspension wird das Rohmaterial unter ständiger Bewegung maximal 3 bis 4 Wochen aufgeschlossen. Anschließend wird das Material durch Säurezugabe neutralisiert und mehrmals gewaschen. Die weitere Aufarbeitung folgt wie üblich. Auf diese Weise lassen sich IEP's von 4 bis 6 einstellen.

Bei Einsatz von Natronlauge läßt sich der Äscherprozeß nochmals verkürzen, wobei bei Konzentrationen von 1 N Natronlauge das Material je nach Zerkleinerungsgrad bereits nach 6 - 12 Stunden aufgeschlossen ist. Die Neutralisation erfolgt mit äquimolaren Mengen Mineralsäure und die Neutralsalze werden durch mehrmaliges Waschen oder durch Entsalzen der in der Extraktion gewonnenen wäßrigen Gelatinelösung entfernt. Bei dieser Verfahrensvariante lassen sich IEP's von 3,5 bis 5 erhalten.

Besonders peptidarme Gelatinesorten werden bei kurzer Verweilzeit im Äscher erhalten. Man kann so Gelatinesorten mit hoher bis mittlerer Molekulargewichtsverteilung ($M = 10^4 - 10^7$ D) erhalten.

Niedrigmolekulare bis kaltwasserlösliche Gelatinesorten kann man durch thermischen Abbau bzw. enzymatisch erhalten.

Bevorzugt werden im Falle der 2-Arylpropionsäurederivate Gelatinesorten mit IEP von 3,5 bis 9,5 eingesetzt.

Übliche pharmazeutische Hilfsstoffe und/oder weitere Makromoleküle können, sofern sie technologisch erforderlich sind, in flüssigem oder getrocknetem Zustand den erfindungsgemäßen Nanosolen zugesetzt werden.

Zum Beispiel kann ein Zusatz von Polyvinylpyrrolidon im Mengenverhältnis Gelatine zu Polyvinylpyrrolidon im Bereich von 5:1 bis 500:1 geeignet sein.

Eine Akutform im Sinne der Erfindung, die z.B. zu Tabletten verarbeitet wird oder lyophilisiert werden soll, kann durch Zusatz von niedrigmolekularen Polyvinylpyrrolidonsorten im Bereich von 10:1 bis 50:1 in den technologischen Verarbeitungseigenschaften verbessert werden, ohne daß die Stabilität der Nanosole negativ beeinflußt wird.

Die in den folgenden Beispielen bevorzugten Herstellungsverfahren, Vorgehensweisen und Bezeichnungen beziehen sich wie folgt auf die deutschen Patentanmeldungen "Pharmazeutisch applizierbares Nanosol und Verfahren zu seiner Herstellung" (P 41 40 195.6) bzw. die oben genannten Verfahren und Beispiele:
Nanosol-Herstellung: Verfahren II und III
Gelatineherstellung: Beispiel I bis III
Vortest: siehe folgende Beschreibung:

Vortest:

Wie eingangs schon erwähnt und wie aus Fig.1 ersichtlich ist, hängt die absolute, maximal mögliche Nettoladung eines einzelnen Gelatinemoleküls hauptsächlich von der Anzahl der freien COOH- und $NH_2$-Gruppen und dem pH-Wert der Lösung ab.

Da sich Typ A, B, Kollagenhydrolysate oder Gelatinederivate in der Anzahl freier COOH-Gruppen unterscheiden, ist damit auch ihre maximal mögliche Nettoladung unterschiedlich. Bei Gelatinederivaten kann der Ladungszustand zusätzlich von der Art der Modifizierung abhängen.

Bei der Durchführung des erfindungsgemäßen Verfahrens wählt man in einem Vortest die geeignete Gelatine und den geeigneten pH-Wert aus.

Zunächst wird ein den physikalisch-chemischen Eigenschaften des Arzneistoffs angepaßter Arbeits-pH-Bereich gewählt. Als physikalisch-chemische Eigenschaft des Arzneistoffs sind vor allem zu berücksichtigen: Die Löslichkeit (in organischen Lösungsmitteln bzw. Wasser), seine Eigenschaft als Säure, Base oder Neutralstoff sowie seine Stabilität gegenüber Säuren und Laugen.

In einem ersten Schnelltest wird festgestellt, welche Ladung die ausgefällten Partikel besitzen. Daraus ergibt sich, unter Berücksichtigung des Arbeits-pH-Bereichs, die Wahl eines geeigneten Gelatinetyps. Sind die Teilchen beispielsweise negativ geladen, sucht man eine Gelatine aus, die unter den gegebenen pH-Bedingungen positiv geladen ist. Dieser Schnelltest zur Feststellung der Partikelladung hat die Vorteile, daß er ohne großen apparativen und zeitlichen Aufwand durchgeführt werden kann. So kann auf eine zeitaufwendige und ungenaue Zeta-Potential-Messung gänzlich verzichtet werden.

In vielen Fällen wird es ausreichend sein, für diesen Schnelltest zwei handelsübliche Gelatinen Typ A und B mit einem IEP von 9,5 bzw. 3,5 mit Peptidanteilen <30 % und einer Bloomzahl von 200, die weiterhin als Standardgelatinen bezeichnet werden, bei einem pH-Wert von 6 in die Solform zu überführen (5%ige wäßrige Lösung) und den Arzneistoff in einem mit Wasser mischbaren Lösungsmittel, wie z. B. Ethanol, Isopropanol oder Aceton, zu lösen und jeweils mit den Gelatinelösungen homogen zu mischen. Bei gleicher Dosierung des Arzneistoffs wird sich bei der in ihrem Ladungszustand nicht geeigneten Gelatine ein kolloidales System entweder nicht ausbilden oder sofort instabil werden bzw. der Arzneistoff ausflocken. Sind die entstehenden Partikel negativ geladen, werden sie eher von Gelatinelösung mit Typ A, der bei einem pH-Wert von 6 positiv geladen ist, stabilisiert als von der Lösung mit Gelatine Typ B; im Gegenteil wird in diesem Fall Typ B entweder kein kolloidales System ausbilden oder das System sofort destabilisieren. Das Ausflocken der Teilchen läßt sich z. B. über eine einfache Trübungs-Messung verfolgen.

Bei diesem Schnelltest muß auf jeden Fall der Arbeits-pH-Bereich beachtet werden. Man kann auch andere Gelatinen als Standard auswählen, sie müssen jedoch in ihrem IEP so gewählt werden, daß sie bei diesem pH-Wert entgegengesetzte Nettoladung tragen (siehe auch Fig.1). In den meisten Fällen werden die besagten Standardgelatinen Typ A und B für diesen Schnelltest ausreichen.

Ausgehend vom Ergebnis des Vorversuchs werden nun durch schrittweise Variation des IEP's durch Verwendung entsprechender Gelatinesorten und des pH-Wertes der Lösung in kleineren Bereichen (z. B. 0,1 pH-Schritte) die optimalen Bedingungen zur Bildung der Nanosole ermittelt. D.h. es muß das Stabilitätsoptimum, das durch den isoionischen Punkt (IIP) gekennzeichnet ist, gefunden werden, um eine ausreichende Stabilität für die genannten pharmazeutischen Anwendungen zu gewährleisten.

Es kann durchaus der Fall sein, daß eine im Sinne der Erfindung akzeptable Stabilität der Nanosole bereits in einem engeren pH-Bereich (ca. 0,5 Einheiten) um den isoionischen Punkt gefunden wird, so daß eine Einstellung dieses Punktes selbst nicht unbedingt notwendig ist. Andererseits können auch mehrere Gelatinen zu den gleichen, stabilen Ergebnissen führen. So kann beispielsweise (Beispiel 5) mit dem oralen Antidiabetikum Glibenclamid bei einem Gelatinetyp B mit einem IEP von 5,5 das Stabilitätsoptimum bei einem pH-Wert von 3,2 liegen, während bei einem Gelatinetyp B mit einem IEP von 3,8 das Stabilitätsoptimum bei einem pH-Wert von 2,2 liegt.

Gekennzeichnet durch ein Stabilitätsmaximum, wurde in beiden Fällen der isoionische Punkt erreicht (die Abhängigkeit der Nettoladung vom pH-Wert und dem IEP muß nicht linear sein, da sie durch den $pK_S$-Wert der vorhandenen COOH- bzw. $NH_3^+$ - Gruppen gegeben ist).

Die Nanosole können beispielsweise sprühgetrocknet werden, wobei ein Zusatz von PVP oder anderer Hilfsstoffe aus technologischer Sicht grundsätzlich möglich ist.

Weiterhin können andere synthetische oder natürliche Makromoleküle zugesetzt werden, sofern sie sich nicht störend auf die Resorption auswirken.

In Abhängigkeit von der Herstellungsweise von Gelatine (Ausmaß des Abbaus des nativen Kollagens und saures bzw. alkalisches Aufschlußverfahren) weist Gelatine vom Typ A oder Typ B ein charakteristisches Molekulargewichtsspektrum bzw. Molekulargewichtsverteilung auf. In Tabelle 1 sind die Molekulargewichtsverteilungen von verschiedenen Gelatinetypen bzw. von Kollagenhydrolysaten angegeben, sowie der prozentuale Anteil (Häufigkeit) einzelner Molekulargewichtsbereiche.

Tabelle 1

| Molekulargewichtsverteilung von verschiedenen bekannten Gelatinetypen bzw. von bekannten Kollagenhydrolysaten | | | | | | | |
|---|---|---|---|---|---|---|---|
| Molecular Mass Distribution (kD) | Native Collagen % | Gelatin Type B % | Gelatin Type A % | Collagen hydrolysate Gelita ® Collagel A | Collagen hydrolysate Gelita ® Collagel B | Collagen hydrolysate Gelita ® Sol C | Elastin hydrolysate Gelita ® Gelastin |
| >360 | 100 | 18,0 | 18,0 | 0 | 0 | 0 | 0 |
| 285 | 0 | 7,0 | 9,0 | 0 | 0 | 0 | 0 |
| 145-237 | 0 | 20,0 | 34,0 | 1,0 | 1,5 | 0 | 0 |
| 95 | 0 | 26,0 | 11,0 | 0 | 0 | 0 | 0 |
| 95-50 | 0 | 16,3 | 13,4 | 2,6 | 4,0 | 1,1 | 0 |
| 50-20 | 0 | 7,4 | 9,1 | 18,0 | 14,5 | 0,3 | 0 |
| 20-10 | 0 | 3,9 | 3,8 | 43,0 | 31,5 | 3,7 | 0,2 |
| 10-5 | 0 | 3,0 | 3,0 | 15,4 | 20,0 | 12,2 | 5,2 |
| 5-2 | 0 | 0 | 0 | 6,0 | 14,0 | 26,0 | 93,9 |
| 2-1 | 0 | 0 | 0 | 7,0 | 8,0 | 23,0 | 0 |
| < 1 | 0 | 0 | 0 | 6,5 | 7,0 | 34,0 | 0 |
| MG | 360 | 165 | 185 | 12-18 | 12-18 | 3 | 2-3 |

Man erkennt in den einzelnen Spalten deutlich das Überwiegen eines einzelnen Bereiches im Vergleich zu den übrigen Molekulargewichtsbereichen derselben Gelatine. Dieser Bereich stellt also das Maximum der Molekulargewichtsverteilung dar (es liegt z.B. bei der in der Abbildung aufgeführten Gelatine Typ B bei 95 kD). Der Begriff des "Maximums der Molekulargewichtsverteilung" ist jedoch streng zu trennen von dem Begriff des "durchschnittlichen mittleren Molekulargewichts". Dieser Mittelwert liegt bei der erwähnten Gelatine vom Typ B bei 165 kD.

Bei der Formulierung von Akut- bzw. Retardpräparaten macht der Pharmazeut einen grundsätzlichen Unterschied zwischen:

1. galenischer Zubereitung, d.h. einer Freisetzung des Arzneistoffes, z.B. aus einer Tablette in zeitlich schneller (Akutform) oder verlangsamter (Retardform) Weise;

und

2. dem arzneistoffspezifischen Resorptionsort, wie z.B. Magen oder bestimmte Darmabschnitte.

Die erfindungsgemäßen Nanosole sind in der Lage, unabhängig von der galenischen Zubereitung, aufgrund ihrer speziellen Zusammensetzung, im gesamten gastrointestinalen Bereich resorbiert zu werden. Sie können daher vorteilhaft zu Akut- bzw. Retardarzneiformen weiterverarbeitet werden.

Wegen der verschiedenen Wirkungsmechanismen für das S- und das R-Enantiomere (S-Flurbiprofen weist in erster Linie antirheumatische Wirkung auf, während das enantiomere R-Flurbiprofen vor allem analgetisch wirkt) können Mischungen aus S- und R-Enantiomeren mit verschiedenen Anteilen der einzelnen Enantiomeren im Einzelfall bevorzugt sein.

Folgende Beispiele sollen die vorliegende Erfindung näher erläutern:

**Beispiel 1:**

Wirkstoff:
S-Flurbiprofen, enantiomerreine Wirkstoffsäure

Gelatinetyp:
handelsüblich Typ B, 280 Bloom (IEP 4,5)
Nanosol-Herstellung:
analog Verfahren II
Gewichtsverhältnis Gelatine/Wirkstoff:
3 :1

600 g oben genannter Gelatine werden in 10 l destilliertem Wasser bei 50 °C aufgelöst. 200 g S-Flurbiprofen werden in 350 g Natronlauge (10%ig) gelöst und der Gelatinelösung zugesetzt. Es wird solange weitergerührt bis eine völlig klare Lösung entsteht. Danach wird durch Zugabe von Salzsäure auf pH 3,2 eingestellt, wobei sich das Nanosol bildet.

Teilchengrößenmessungen (BI-FOQUELS On-line Particle-Sizer) ergeben zu 80% Teilchengrößen des Nanosols kleiner als 410 nm.

Durch anschließende Sprühtrocknung wird das Wasser entzogen. Das getrocknete Nanosol wird auf einer Exzenterpresse zu Retard-Matrixtabletten mit jeweils 200 mg S-Flurbiprofengehalt verpreßt.

Der Dissolutiontest (900 ml, pH 6,5, 100 Upm) ergibt eine nahezu lineare Freigabe, bei der 100% innerhalb 8 Stunden freigesetzt werden.

**Beispiel 2:**

Wirkstoff:
S-Flurbiprofen, enantiomerreine Wirkstoffsäure
Gelatinetyp:
Typ B, 330 Bloom (IEP 4,5), Herstellung Beispiel II, vollentsalzt
Nanosol-Herstellung:
analog Verfahren III
Gewichtsverhältnis Gelatine/Wirkstoff:
1:1

200 g oben genannter Gelatine werden in 4 l destilliertem Wasser bei 60°C gelöst. Mit Salzsäure wird auf einen pH-Wert von 3,2 eingestellt. 200 g S-Flurbiprofen werden in 700 ml Ethanol gelöst. Die alkoholische Lösung wird mit der Gelatinelösung vereinigt, wobei sich das Nanosol bildet.

Das organische Lösungsmittel wird unter Vakuum entfernt und die Lösung wie in Beispiel 1 sprühgetrocknet.

Das getrocknete Nanosol wird granuliert und auf einer Exzenterpresse zu Retard-Matrixtabletten mit jeweils 200 mg S-Flurbiprofengehalt verpreßt.

**Beispiel 3:**

a) R-Flurbiprofen Akut-Nanosol

Wirkstoff:
R-Flurbiprofen, enantiomerreine Wirkstoffsäure
Gelatinetyp:
Typ B (IEP 4,5), kaltwasserlöslich, Herstellung Beispiel II
Nanosol-Herstellung:
analog Verfahren III
Gewichtsverhältnis Gelatine/Wirkstoff:
2:1

Die Mengenverhältnisse und Herstellungsbedingungen sind wie folgt:
Gelatine: 300 g in 3 l destilliertem Wasser von Raumtemperatur gelöst, eingestellt auf pH 3,2.

R-Flurbiprofen: 150 g, gelöst in 500 ml Alkohol.

Nach Entfernung des Alkohols unter Vakuum wird das Nanosol sprühgetrocknet und trockengranuliert.

b) S-Flurbiprofen Retard-Nanosol

Analog Beispiel 2.

Aus a) und b) wird eine Akut- und Retardform wie folgt gebildet:
Eine Retardtablette aus a) wird zusammen mit jeweils 75 mg des in b) hergestellten Granulats in Hartgelatinekapseln abgefüllt. Die S-Flurbiprofen-Dosis beträgt 200 mg und die R-Flurbiprofen-Initialdosis 25 mg.

## Patentansprüche

1. Retard-Arzneimittel zur Behandlung von schmerzhaften und/oder entzündlichen, sowie fieberhaften Erkrankungen, enthaltend Flurbiprofen neben üblichen pharmazeutischen Trägern und Hilfsstoffen, dadurch gekennzeichnet, daß das Flurbiprofen in Form eines pharmazeutisch applizierbaren Nanosols vorliegt, das als Träger im wesentlichen Gelatine, fraktionierte Gelatine oder ein Gelatinederivat enthält, wobei das Nanosol und der Träger einen annähernden oder vollständigen isoionischen Ladungsausgleich aufweisen.

2. Arzneimittel nach Anspruch 1, dadurch gekennzeichnet, daß das Flurbiprofen als Racemat vorliegt.

3. Arzneimittel nach Anspruch 1, dadurch gekennzeichnet, daß das Flurbiprofen als Pseudoracemat oder als Gemisch aus dem Racemat mit seinen Enantiomeren vorliegt.

4. Arzneimittel nach Anspruch 1, dadurch gekennzeichnet, daß das Flurbiprofen als reines S- oder R-Enantiomer vorliegt.

5. Arzneimittel nach Anspruch 1, dadurch gekennzeichnet, daß das Flurbiprofen als Gemisch unterschiedlicher Anteile an S- und R-Enantiomeren vorliegt.

6. Arzneimittel nach Anspruch 3, dadurch gekennzeichnet, daß das Flurbiprofen als Pseudoracemat, d.h. in Form einer Mischung der zuvor separierten Enantiomeren vorliegt.

7. Arzneimittel nach einem der Ansprüche 1 bis 6, dadurch gekennzeichnet, daß das Nanosol

   a) eine innere Phase aus dem Flurbiprofen, das eine Teilchengröße von 10 - 800 nm aufweist und eine Oberflächenladung besitzt,
   b) eine äußere Phase aus Gelatine, fraktionierter Gelatine oder einem Gelatinederivat, welche(s) gegensinnig geladen ist, und
   c) einen annähernden oder vollständigen isoionischen Ladungszustand der inneren und äußeren Phase aufweist und
   d) physiologisch resorbierbar ist.

8. Arzneimittel nach einem der Ansprüche 1 bis 7, dadurch gekennzeichnet, daß das Flurbiprofen als feste, resuspendierbare Nanodispersion vorliegt.

9. Arzneimittel nach Anspruch 7 und/oder 8, dadurch gekennzeichnet, daß das Flurbiprofen eine durchschnittliche Teilchengröße unterhalb von 400 nm aufweist.

10. Arzneimittel nach einem der Ansprüche 1 - 9, dadurch gekennzeichnet, daß die Gelatine ein Maximum der Molekulargewichtsverteilung im Bereich von $10^4$ bis $10^7$ D aufweist.

11. Arzneimittel nach Anspruch 10, dadurch gekennzeichnet, daß die Gelatine ein Maximum der Molekulargewichtsverteilung oberhalb von $9,5 \times 10^4$ D und einen Peptidanteil kleiner als 5 Gewichtsprozent aufweist.

12. Arzneimittel nach einem der Ansprüche 1 - 11, dadurch gekennzeichnet, daß die Gelatine einen prozentualen Gewichtsanteil der Mikrogelfraktion (> $10^7$ D) größer als 15 % Prozent aufweist.

13. Arzneimittel nach einem der Ansprüche 1 bis 12, gekennzeichnet durch eine äußere Phase des Nanosols, die zusätzlich viskositätserhöhende Stoffe in einem Gewichtsverhältnis von Gelatine zu synthetischem oder natürlichem Polymer wie 10:1 bis 1000:1 enthält.

14. Arzneimittel nach einem der Ansprüche 1-13, dadurch gekennzeichnet, daß die Retardform eine Tablette ist.

15. Arzneimittel nach Anspruch 14, dadurch gekennzeichnet, daß die Retardform eine Matrixtablette ist.

16. Arzneimittel zur Behandlung von schmerzhaften und/oder entzündlichen, sowie fieberhaften Erkrankungen, enthaltend Flurbiprofen neben üblichen pharmazeutischen Trägern und Hilfsstoffen, dadurch gekennzeichnet, daß es teilweise als Akutform, teilweise als Retardform mit Flurbiprofen in Form eines pharmazeutisch applizierbaren Nanosols vorliegt.

17. Arzneimittel nach Anspruch 16, dadurch gekennzeichnet, daß die Akut- und Retardform in einer Hartgelatinekapsel vorliegen.

18. Arzneimittel nach einem der Ansprüche 1-16, dadurch gekennzeichnet, daß das Flurbiprofen zu mehr als 50% als S- oder R-Flurbiprofen vorliegt.

19. Verwendung eines pharmazeutisch applizierbaren Nanosols von Flurbiprofen zur Herstellung von Arzneimitteln mit analgetischer und/oder antirheumatischer Retardwirkung.

20. Verwendung eines pharmazeutisch applizierbaren Nanosols von Flurbiprofen zur Herstellung von Arzneimitteln mit analgetischer und/oder antirheumatischer, sowie antipyretischer Akut- und Retardwirkung.

21. Verwendung nach Anspruch 19 oder 20, dadurch gekennzeichnet, daß das Flurbiprofen zu mehr als 50 % als S- oder R-Flurbiprofen vorliegt.

## Claims

1. Controlled-release medicament for the treatment of painful and/or inflammatory, and also febrile disorders, containing flurbiprofen in addition to customary pharmaceutical excipients and auxiliaries, characterized in that the flurbiprofen is present in the form of a pharmaceutically administrable nanosol which, as excipient, essentially contains gelatin, fractionated gelatin or a gelatin derivative, the nanosol and the excipient having an approximate or complete isoionic charge equalization.

2. Medicament according to Claim 1, characterized in that the flurbiprofen is present as a racemate.

3. Medicament according to Claim 1, characterized in that the flurbiprofen is present as a pseudoracemate or as a mixture of the racemate with its enantiomers.

4. Medicament according to Claim 1, characterized in that the flurbiprofen is present as a pure S- or R-enantiomer.

5. Medicament according to Claim 1, characterized in that the flurbiprofen is present as a mixture of different proportions of S- and R-enantiomers.

6. Medicament according to Claim 3, characterized in that the flurbiprofen is present as a pseudoracemate, i.e. in the form of a mixture of the previously separated enantiomers.

7. Medicament according to one of Claims 1 to 6, characterized in that the nanosol

   a) possesses an inner phase of the flurbiprofen, which has a particle size of 10-800 nm and a surface charge,
   b) has an outer phase of gelatin, fractionated gelatin or a gelatin derivative, which is oppositely charged, and
   c) an approximate or complete isoionic charge state of the inner and outer phases and
   d) is physiologically absorbable.

8. Medicament according to one of Claims 1 to 7, characterized in that the flurbiprofen is present as a solid, resuspendable nanodispersion.

9. Medicament according to Claim 7 and/or 8, characterized in that the flurbiprofen has an average particle size of below 400 nm.

10. Medicament according to one of Claims 1-9, characterized in that the gelatin has a maximum of the molecular weight distribution in the range from $10^4$ to $10^7$D.

11. Medicament according to Claim 10, characterized in that the gelatin has a maximum of the molecular weight distribution of above $9.5 \times 10^4$D and a peptide content of less than 5% by weight.

12. Medicament according to one of Claims 1-11, characterized in that the gelatin has a percentage weight content of the microgel fraction (> $10^7$ D) of greater than 15%.

13. Medicament according to one of Claims 1 to 12, characterized by an outer phase of the nanosol which additionally contains viscosity-enhancing substances in a weight ratio of gelatin to synthetic or natural polymer such as 10:1 to 1000:1.

14. Medicament according to one of Claims 1-13, characterized in that the controlled-release form is a tablet.

15. Medicament according to Claim 14, characterized in that the controlled-release form is a matrix tablet.

16. Medicament for the treatment of painful and/or inflammatory, and also febrile disorders, containing flurbiprofen in addition to customary pharmaceutical excipients and auxiliaries, characterized in that it is partially present as an immediate-effect form and partially as a controlled-release form containing flurbiprofen in the form of a pharmaceutically administrable nanosol.

17. Medicament according to Claim 16, characterized in that the immediate-effect and controlled-release forms are present in a hard gelatin capsule.

18. Medicament according to one of Claims 1-16, characterized in that the flurbiprofen is present to more than 50% as S- or R-flurbiprofen.

19. Use of a pharmaceutically administrable nanosol of flurbiprofen for the production of medicaments having analgesic and/or antirheumatic controlled-release action.

20. Use of a pharmaceutically administrable nanosol of flurbiprofen for the production of medicaments having analgesic and/or antirheumatic, and also anti pyretic immediate-effect and controlled-release actions.

21. Use according to Claim 19 or 20, characterized in that the flurbiprofen is present to more than 50% as S- or R-flurbiprofen.

**Revendications**

1. Médicament à effet retardé destiné au traitement de maladies accompagnées de douleur et/ou d'inflammation ainsi que de fièvre, contenant du flurbiprofène à côté de supports et de substances auxiliaires pharmaceutiques classiques, caractérisé en ce que le flurbiprofène est présent sous forme d'un nanosol pharmaceutiquement applicable, qui contient comme support principalement de la gélatine, de la gélatine fractionnée ou un dérivé de gélatine, le nanosol et le support présentant un équilibre de charge isoionique approximatif ou total.

2. Médicament suivant la revendication 1, caractérisé en ce que le flurbiprofène est présent sous forme du racémate.

3. Médicament suivant la revendication 1, caractérisé en ce que le flurbiprofène est présent sous forme du pseudoracémate ou comme mélange du racémate avec ses énantiomères.

4. Médicament suivant la revendication 1, caractérisé en ce que le flurbiprofène est présent sous forme de l'énantiomère S ou R pur.

5. Médicament suivant la revendication 1, caractérisé en ce que le flurbiprofène est présent sous forme de mélange de différentes proportions des énantiomères S et R.

6. Médicament suivant la revendication 3, caractérisé en ce que le flurbiprofène est présent sous forme du pseudoracémate, c'est-à-dire sous forme d'un mélange des énantiomères préalablement séparés.

7. Médicament suivant l'une des revendications 1 à 6, caractérisé en ce que le nanosol

a) présente une phase interne du flurbiprofène, qui a des diamètres de particules de 10 à 800 nm et qui possède une charge de surface,
b) présente une phase externe de gélatine, de gélatine fractionnée ou d'un dérivé de gélatine qui porte une charge opposée, et
c) présente un état de charge isoionique approximatif ou total de la phase interne et de la phase externe et
d) peut être résorbé physiologiquement.

8. Médicament suivant l'une des revendications 1 à 7, caractérisé en ce que le flurbiprofène se présente comme nanodispersion solide pouvant être remise en suspension.

9. Médicament suivant la revendication 7 et/ou la revendication 8, caractérisé en ce que le flurbiprofène présente un diamètre moyen de particules inférieur à 400 nm.

10. Médicament suivant l'une des revendications 1 à 9, caractérisé en ce que la gélatine présente un maximum de répartition de poids moléculaire dans la plage de $10^4$ à $10^7$ D.

11. Médicament suivant la revendication 10, caractérisé en ce que la gélatine présente un maximum de répartition de poids moléculaire au-dessus de $9,5x10^4$ D et une proportion de peptide inférieure à 5 % en poids.

12. Médicament suivant l'une des revendications 1 à 11, caractérisé en ce que la gélatine présente un pourcentage en poids de fraction de microgel (> $10^7$ D) de plus de 15 % en poids.

13. Médicament suivant l'une des revendications 1 à 12, caractérisé par une phase externe du nanosol qui contient en outre des substances élevant la viscosité dans un rapport en poids de la gélatine avec le polymère synthétique ou naturel de 10:1 à 1000:1.

14. Médicament suivant l'une des revendications 1 à 13, caractérisé en ce que la forme retardée est un comprimé.

15. Médicament suivant la revendication 14, caractérisé en ce que la forme retardée est un comprimé à matrice.

16. Médicament destiné au traitement de maladies accompagnées de douleur et/ou d'inflammation ainsi que de fièvre, contenant du flurbiprofène à côté de supports et de substances auxiliaires pharmaceutiques classiques, caractérisé en ce qu'il se présente en partie comme forme à action rapide et en partie comme forme à action retardée avec du flurbiprofène sous forme d'un nanosol pharmaceutiquement applicable.

17. Médicament suivant la revendication 16, caractérisé en ce que la forme à action rapide et la forme retardée sont présentes dans une gélule de gélatine dure.

18. Médicament suivant l'une des revendications 1 à 16, caractérisé en ce que le flurbiprofène est présent en proportion de plus de 50 % comme flurbiprofène S ou R.

19. Utilisation d'un nanosol de flurbiprofène pharmaceutiquement applicable pour la préparation de médicaments à action analgésique et/ou antirhumatismale retardée.

20. Utilisation d'un nanosol de flurbiprofène pharmaceutiquement applicable pour la préparation de médicaments à action analgésique et/ou antirhumatismale ainsi qu'antipyrétique rapide et retardée.

21. Utilisation suivant la revendication 19 ou 20, caractérisée en ce que le flurbiprofène est présent en proportion de plus de 50 % comme flurbiprofène S ou R.

Fig.1

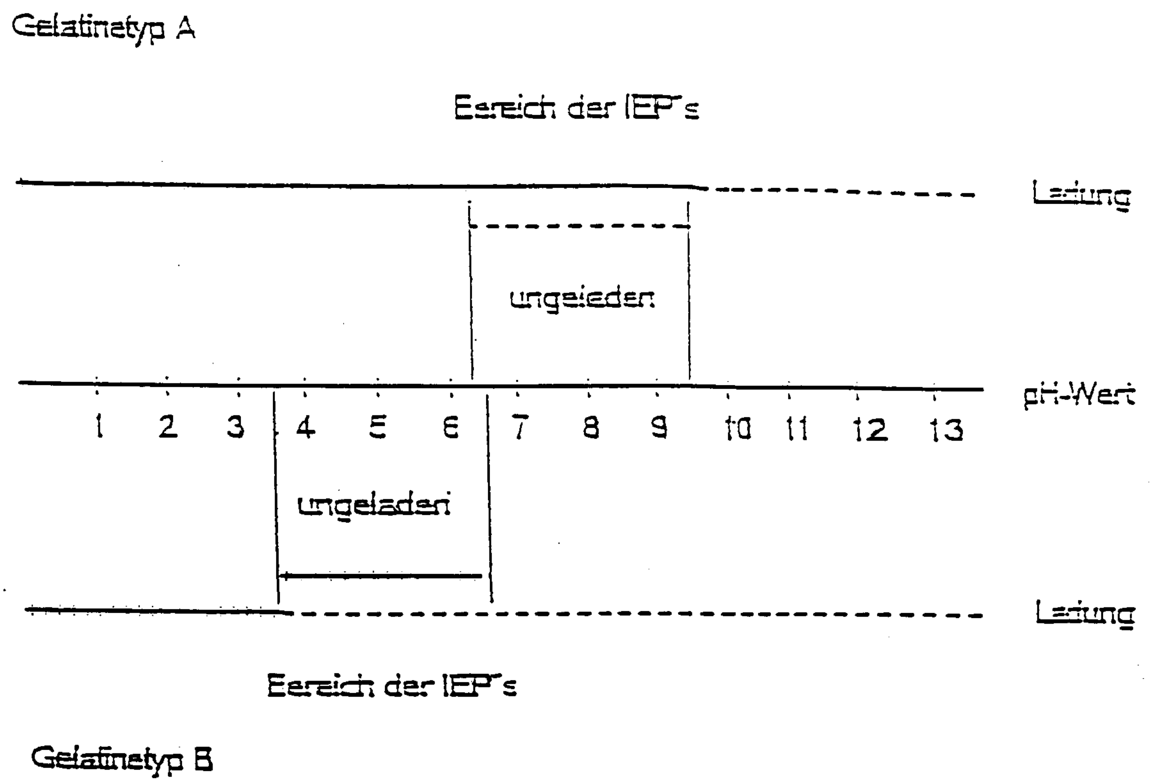


Ladungsverteilungen in den Gelatinetypen A (sauer) und B (alkalisch)

IEP = isoelektrischer Punkt

Fig. 2

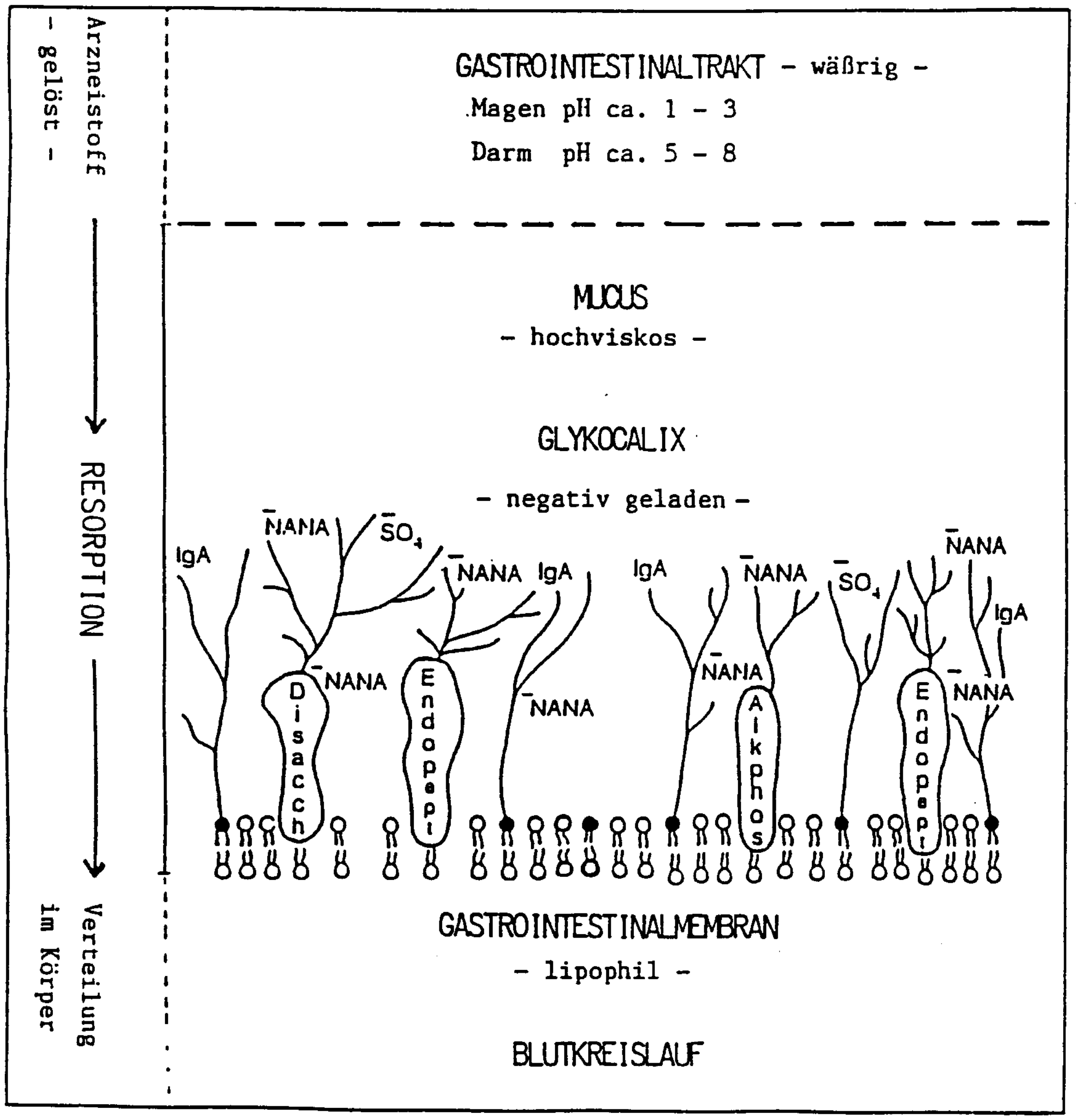
Arzneistoff
- gelöst -
RESORPTION
Verteilung
im Körper
GASTROINTESTINALTRAKT - wäßrig -
Magen pH ca. 1 - 3
Darm pH ca. 5 - 8
MUCUS
- hochviskos -
GLYKOCALIX
- negativ geladen -
IgA
NANA
SO4
Disacch
Endopept
Alkphos
GASTROINTESTINALMEMBRAN
- lipophil -
BLUTKREISLAUF